# FASCICULE DE BREVET EUROPEEN

(11) **EP 3 544 951 B1**
(45) Date de publication et mention de la délivrance du brevet: **05.05.2021**
(21) Numéro de dépôt: 17811492.2
(22) Date de dépôt: 23.11.2017
(51) Int. Cl.: C07C 69/732, A61K 31/216, A61K 31/25, A61P 25/28

(54) **DERIVES D'ACIDES HEXARIQUES SUBSTITUES ET LEURS UTILISATIONS**
SUBSTITUIERTE HEXARINSÄUREDERIVATE UND VERWENDUNGEN DAVON
SUBSTITUTED HEXARIC ACID DERIVATIVES AND USES THEREOF

(30) Priorité: 25.11.2016 FR 1661487
(43) Date de publication de la demande: 02.10.2019
(73) Titulaire: Plant Advanced Technologies Pat, 54500 Vandoeuvre-les-Nancy (FR)
(72) Inventeur: WARNAULT, Pierre, 54360 Blainville sur l'Eau (FR)
(74) Mandataire: IPAZ
(86) Numéro de dépôt international: PCT/EP2017/080226
(87) Numéro de publication internationale: WO 2018/096040

(56) Documents cités:
- CN-A- 101 292 972
- CN-A- 101 486 743
- CN-A- 101 856 347
- JP-B1- 3 039 864
- SCHWAIGER S ET AL: "Leontopodic acid-a novel highly substituted glucaric acid derivative from Edelweiss (Leontopodium alpinum Cass.) and its antioxidative and DNA protecting properties", TETRAHEDRON, ELSEVIER SCIENCE PUBLISHERS, AMSTERDAM, NL, vol. 61, no. 19, 9 mai 2005 (2005-05-09), pages 4621-4630, XP027862041, ISSN: 0040-4020 [extrait le 2005-05-09] cité dans la demande
- DUDEK MARTA K ET AL: "Caffeic acid derivatives isolated from the aerial parts ofGalinsoga parvifloraand their effect on inhibiting oxidative burst in human neutrophils", PHYTOCHEMISTRY LETTERS, ELSEVIER, AMSTERDAM, NL, vol. 16, 4 juin 2016 (2016-06-04), pages 303-310, XP029601939, ISSN: 1874-3900, DOI: 10.1016/J.PHYTOL.2016.05.007 cité dans la demande
- TETSUYA SENGOKU ET AL: "Synthesis of novel mucic acid 1,4-lactone methyl ester 3--ferulate related to an extractive component isolated from the peels of", TETRAHEDRON LETTERS, ELSEVIER, AMSTERDAM, NL, vol. 53, no. 4, 14 novembre 2011 (2011-11-14), pages 435-437, XP028394809, ISSN: 0040-4039, DOI: 10.1016/J.TETLET.2011.11.066 [extrait le 2011-11-22]

## Description

### DOMAINE DE L'INVENTION

La présente invention concerne des dérivés d'acides hexariques polysubstitués, notamment des acides polycaféoylhexariques (PCH), et leurs dérivés ester. L'invention concerne également l'utilisation de ces composés en tant que médicament, notamment pour la prévention et/ou le traitement des maladies neurodégénératives, notamment de la maladie d'Alzheimer, et les compositions pharmaceutiques ou nutraceutiques les contenant.

### ART ANTERIEUR

Un dérivé de l'acide glucarique entièrement substitué, appelé acide léontopodique, a été identifié comme l'un des principaux composés des parties aériennes de l'Edelweiss, il présente un pouvoir antioxydant prononcé, comparable à celui de l'acide caféique (S. Schwaiger et al., Leontopodic acid-a novel highly substituted glucaric acid derivative from Edelweiss (Leontopodium alpinum Cass.) and its antioxidative and DNA protecting properties. Tetrahedron, Volume 61, Issue 19, 2005, 4621-4630).

Dans la demande CN101856347, l'acide léontopodique extrait d'une plante du genre Edelweiss et une composition pharmaceutique contenant ce composé présentent des effets sur la résistance au virus de l'hépatite, ont des propriétés anti-inflammatoires, protègent le foie, régulent l'immunité et ainsi peuvent- être utilisés pour traiter l'hépatite.

L'article de Costa *et al.* (2010) décrit le rôle protecteur d'un prétraitement de cellules avec de l'acide léontopodique isolé d'une plante du genre Edelweiss : étonnamment, en présence de ce composé aucune amélioration de la survie cellulaire n'est observée bien que la production d'espèces réactives de l'oxygène (Reactive Oxygen Species, ou ROS) soit diminuée (S. Costa et al., Free radicals and antioxidants in two oxidative-stress cell models exposed to ochratoxin A and amyloid β : unexpected results. World Mycotoxin journal, August 2010, 3(3), 257-261).

L'article de Lulli *et al.* (Lulli D. et al., Anti-inflammatory effects of concentrated ethanol extracts of Edelweiss (Leontopodim alpinum cass.) callus structures towards human keratinocytes and endothelial cells. Mediators of Inflammation, vol. 2012, article ID 498373) décrit l'effet anti-inflammatoire d'extraits éthanoliques d'Edelweiss comprenant de l'acide léontopodique.

L'article de Dudek *et al.* décrit l'effet anti-oxydatif exercé sur des neutrophiles par des dérivés d'acide hexarique (Dudek et al., Caffeic acid derivatives isolated from the aerial parts of Galinsoga parviflora and their effect on inhibiting oxidative burst in human neutrophils. Phytochemistry Letters 16 (2016), 303-310).

De manière surprenante, les inventeurs ont maintenant mis en évidence que des acides hexariques polysubstitués, notamment des acides polycaféoylhexariques, en particulier l'acide léontopodique, et plus particulièrement l'acide léontopodique B et certains de ses dérivés esters, présentent des propriétés neuroprotectrices, et notamment des capacités significatives dans la prévention et/ou le traitement des maladies neurodégénératives, en particulier de la maladie d'Alzheimer.

### DESCRIPTION DES FIGURES

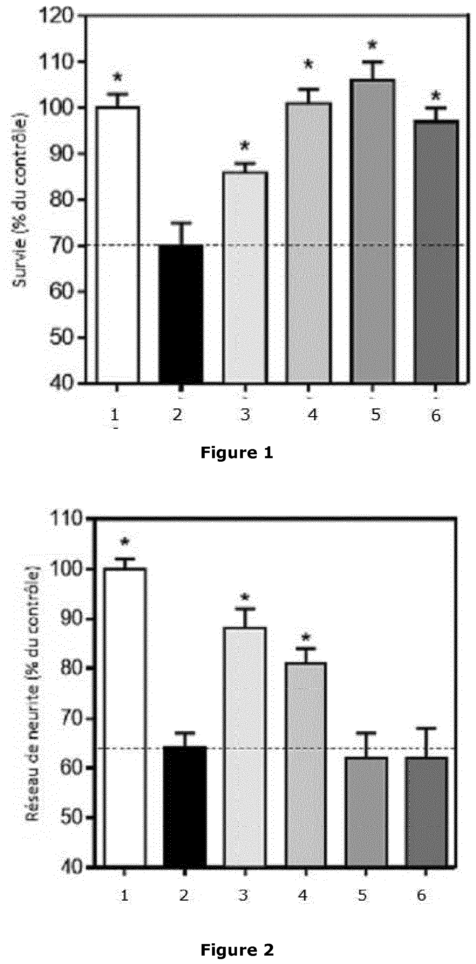

La Figure 1 montre la survie, en pourcentage de neurones vivants par rapport au contrôle, de neurones corticaux primaires intoxiqués pendant 24 heures par le peptide Aβ 1-42 ajouté à une concentration finale de 20 µM, en présence d'acide léontopodique B1 (ALB1) à des concentrations variant de 10 nM à 10 µM conformément à l'exemple 1. Les barres de l'histogramme représentent : témoin contrôle (1), + peptide Aβ 1-42 (2), peptide Aβ 1-42 + ALB1 10 nM (3), peptide Aβ 1-42 + ALB1 100 nM (4), peptide Aβ 1-42 + ALB1 1 µM (5), peptide Aβ 1-42 + ALB1 10 µM (6).
La Figure 2 montre la croissance du réseau de neurites intoxiqués pendant 24 heures par le peptide Aβ 1-42 ajouté à une concentration finale de 20 µM, en présence d'acide léontopodique B1 à des concentrations variant de 10 nM à 10 µM conformément à l'exemple 1. Les barres de l'histogramme représentent: contrôle (1), + peptide Aβ 1-42 (2), peptide Aβ 1-42 + ALB1 10 nM (3), peptide Aβ 1-42 + ALB1 100 nM (4), peptide Aβ 1-42 + ALB1 1 µM (5), peptide Aβ 1-42 + ALB1 10 µM (6).

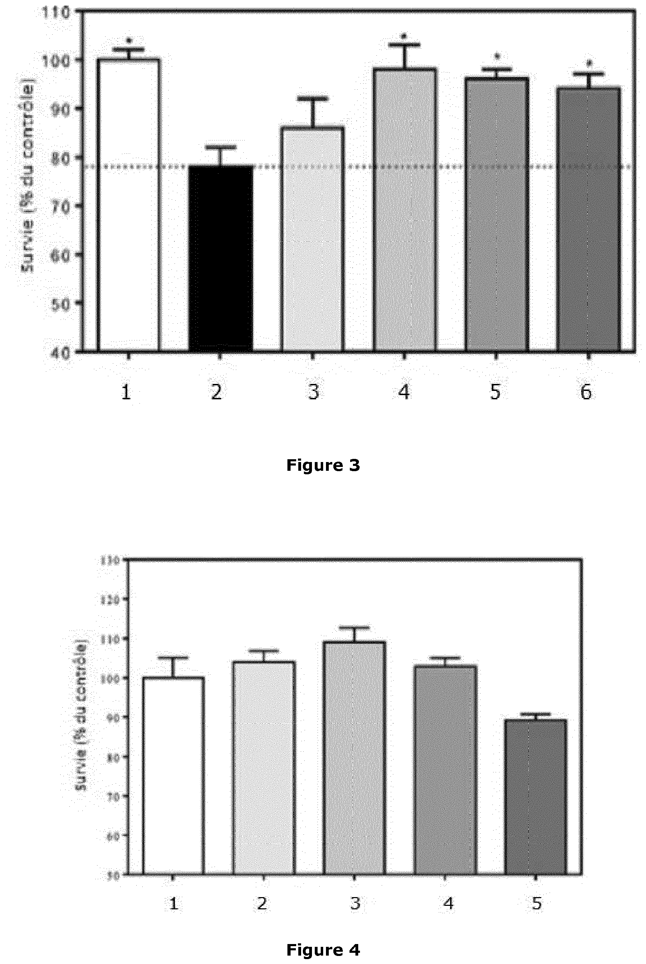

La Figure 3 montre le pourcentage de survie des astrocytes par rapport au contrôle dans une culture de neurones corticaux primaires intoxiqués pendant 24 heures par le peptide Aβ 1-42 ajouté à une concentration finale de 20 µM, en présence d'acide léontopodique B1 à des concentrations variant de 10 nM à 10 µM conformément à l'exemple 1. Les barres représentent, de gauche à droite : témoin contrôle (1), + peptide Aβ 1-42 (2), peptide Aβ 1-42 + ALB1 10 nM (3), peptide Aβ 1-42 + ALB1 100 nM (4), peptide Aβ 1-42 + ALB1 1 µM (5), peptide Aβ 1-42 + ALB1 10 µM (6).

La Figure 4 montre le pourcentage de survie des astrocytes par rapport au contrôle dans une culture pure d'astrocytes sains et non intoxiqués par le peptide Aβ 1-42, en présence d'acide léontopodique B1 à des concentrations de 100 nM, 1 µM, 10 µM et 50 µM, conformément à l'exemple 2. Les barres représentent, de gauche à droite : contrôle sans ALB1 (1), avec ALB1 100 nM (2), avec ALB1 1 µM (3), avec ALB1 10 µM (4), avec ALB1 50 µM (5).

### DESCRIPTION DETAILLEE DE L'INVENTION

Dans un premier aspect, la présente invention est relative à des composés de formule générale (I) : 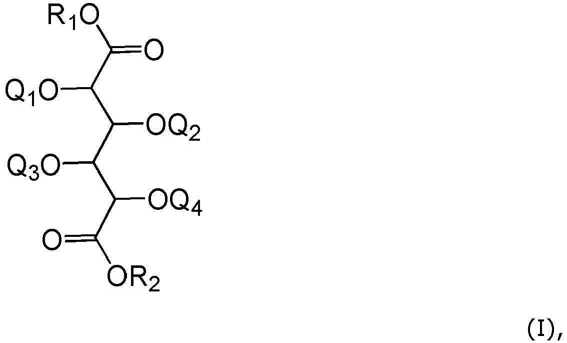 dans laquelle
R₁ et R₂ sont identiques ou différents et représentent, chacun indépendamment l'un de l'autre, un atome d'hydrogène ou un groupe alkyle en C₁-Cₚ linéaire ou ramifié, cyclique ou cyclique ramifié, saturé ou insaturé, pouvant être substitué par un ou plusieurs substituants choisis dans le groupe comprenant : -OR₃, - NR₃R₄, -NO₂, -COR₃, -COOR₃, -CF₃, -CR₃F₂, -CR₃R₄F, =O, -CN, halogène, -SR₃, - SO₂R₃ et aryle, avec
   - p est un entier naturel compris entre 2 et 18,
   - R₃ et R₄ sont identiques ou différents et représentent, chacun indépendamment l'un de l'autre,
      - Soit un groupe choisi dans le groupe comprenant : -H, -OH, - NH₂, -NO₂, -COOH, -CF₃, -CHF₂, -CH₂F, =O, -CN, halogène, - SH et - SO₂H,
      - Soit un groupe alkyle en C₁-C₆ linéaire ou ramifié, cyclique ou cyclique ramifié, saturé ou insaturé, pouvant être substitué par un ou plusieurs substituants choisis dans le groupe comprenant : -H, -OH, -NH₂, -NO₂, -COOH, -CF₃, -CHF₂, -CH₂F, =O, -CN, halogène, -SH et -SO₂H,
   et
Q₁, Q₂, Q₃ et Q₄ représentent, chacun indépendamment l'un de l'autre, un radical choisi dans le groupe comprenant :
   un groupe caféoyle, un groupe CH₃-CH(OH)-CH₂-CO- et un atome d'hydrogène, sous réserve qu'au moins un de ces radicaux soit un groupe caféoyle ; leurs stéréoisomères et leurs sels pharmaceutiquement ou nutraceutiquement acceptables, pour leur utilisation dans la prévention et/ou le traitement d'une maladie neurodégénérative.

Les composés de formule générale (I) peuvent être utilisés seuls ou sous forme d'un mélange de composés de formule (I).

Par « groupe alkyle en C₁-Cₚ », on entend, au sens de la présente invention, une chaîne hydrocarbonée monovalente saturée ou insaturée, linéaire ou ramifiée, cyclique ou cyclique ramifiée, comportant de 1 à p atomes de carbone, p étant un entier naturel compris entre 2 et 18.

Conformément à l'invention, R₁ et R₂ représentent, chacun indépendamment, un atome d'hydrogène ou une chaîne hydrocarbonée monovalente saturée ou insaturée, linéaire ou ramifiée, cyclique ou cyclique ramifiée de 1 à 18 atomes de carbones, de préférence de 1 à 17 atomes de carbone, et plus préférentiellement de 1 à 16 atomes de carbone, de 1 à 15 atomes de carbone, de 1 à 14 atomes de carbone, de 1 à 13 atomes de carbone, de 1 à 12 atomes de carbone, de 1 à 11 atomes de carbone, de 1 à 10 atomes de carbone, de 1 à 9 atomes de carbone, de 1 à 8 atomes de carbone, de 1 à 7 atomes de carbone, de préférence de 2, 3, 4, 5 ou 6 atomes de carbone, et en particulier de 2, 5 ou 6 atomes de carbone.

A titre d'exemple et de façon non exhaustive, on peut citer les groupes éthyle, n-propyle, isopropyle, n-butyle, isobuyle, tert-butyle, pentyle, hexyle, heptyle, octyle, nonyle, décyle, undécyle, dodécyle, tridécyle, tétradécyle, pentadécyle, hexadécyle, heptadécyle, octadécyle, géranyle, farnésyle, cyclopropyle, cyclobutyle, cyclohexyle, éthylényle, propylényle, butylényle, cyclopropènyle, cyclobutènyle, cylcopentènyle, cyclohexènyle, cycloheptènyle, cyclohexa-1,3-diènyle, cyclohexa-1,4-diènyle, cyclohexa-1,5-diènyle, lesdits groupes pouvant être linéaires ou ramifiés.

Dans un mode de réalisation préférée de l'invention, les groupes alkyles sont linéaires.

Par « groupe aryle », on entend un cycle aromatique en C₆, pouvant être substitué par un ou plusieurs substituants choisis dans le groupe comprenant : -H, -OH, -NH₂, -NO₂, -COOH, -CF₃, -CHF₂, -CH₂F, =O, -CN, halogène, -SH et - SO₂H.

Par « groupe caféoyle », on entend un radical de formule (II), issu de l'acide caféique :

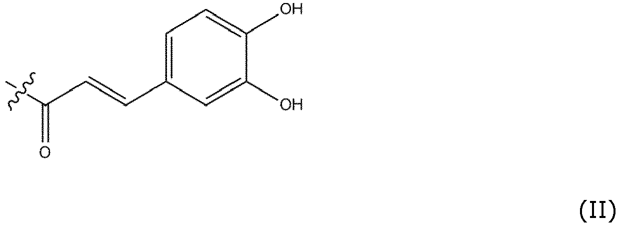

Par « groupe CH₃-CH(OH)-CH₂-CO- », on entend un radical 3-hydroxy-butanoyl.

Par « acide hexarique polysubstitué », on entend un composé répondant à la formule générale (III) suivante : 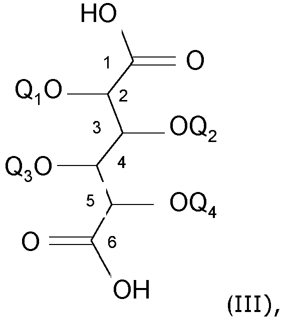 dans laquelle Q₁, Q₂, Q₃ et Q₄ représentent, chacun indépendamment l'un de l'autre, un radical choisi dans le groupe comprenant : un groupe caféoyle, un groupe 3-hydroxybutanoyl de formule CH₃-CH(OH)-CH₂-CO- et un atome d'hydrogène, sous réserve qu'au moins un de ces radicaux soit un groupe caféoyle.

Par « acide polycaféoylhexarique (PCH) » on entend un composé répondant à la formule générale (III) ci-dessus, dans laquelle au moins deux quelconques des radicaux Q₁, Q₂, Q₃ et Q₄ représentent un groupe caféoyle, et notamment on entend un composé répondant à la formule générale (III) ci-dessus dans laquelle trois quelconques des radicaux Q₁, Q₂, Q₃ et Q₄ représentent un groupe caféoyle.

Par « acide glucarique » on entend un acide hexarique répondant à la formule générale (III) ci-dessus dans laquelle, respectivement, les substituant -OQ₁ du carbone numéro 2, -OQ₂ du carbone numéro 3 et -OQ₃ du carbone numéro 4 sont situés au-dessus du plan et le substituant -OQ₄ du carbone numéro 5 est situé au-dessous du plan.

Par « acide altrarique » on entend un acide hexarique répondant à la formule générale (III) ci-dessus dans laquelle, respectivement, les substituants -OQ₁ du carbone numéro 2, -OQ₃ du carbone numéro 4 et -OQ₄ du carbone numéro 5 sont situés au-dessus du plan et le substituant -OQ₂ du carbone numéro 3 est situé au-dessous du plan.

Par « acide léontopodique B » on entend un acide hexarique répondant à la formule générale (III) ci-dessus dans laquelle trois quelconques des substituants Q₁, Q₂, Q₃ et Q₄ représentent un groupe caféoyle et le quatrième radical représente un atome d'hydrogène.

Par « acide léontopodique B1 » on entend l'acide léontopodique B répondant à la formule générale (III) ci-dessus dans laquelle les substituants Q₂, Q₃ et Q₄ représentent un groupe caféoyle et le radical Q₁ représente un atome d'hydrogène. L'acide léontopodique B1 a été identifié la première fois dans les parties aériennes de *Leontopodium alpinum* (Edelweiss) (S. Schwaiger et al. ; Development of an HPLC-PAD-MS assay for the identification and quantification of major phenolic edelweiss (Leontopodium alpium Cass.) constituents, Phytochemical Analysis, volume 17, no. 5, 2006, 291-298).

Parmi les composés de formule (I) pour leur utilisation selon la présente invention, on peut citer les acides suivants :
- L'acide 2 caféoylhexarique (Q₁ = radical caféoyle, Q₂ = Q₃ = Q₄ = H)
- L'acide 3 caféoylhexarique (Q₂ = radical caféoyle, Q₁ = Q₃ = Q₄ = H)
- L'acide 4 caféoylhexarique (Q₃ = radical caféoyle, Q₁ = Q₂ = Q₃ = H)
- L'acide 5 caféoylhexarique (Q₄ = radical caféoyle, Q₁ = Q₂ = Q₃ = H)
- L'acide 2,3 dicaféoylhexarique (Q₁ = Q₂ = radical caféoyle, Q₃ = Q₄ = H)
- L'acide 2,4 dicaféoylhexarique (Q₁ = Q₃ = radical caféoyle, Q₂ = Q₄ = H)
- L'acide 2,5 dicaféoylhexarique (Q₁ = Q₄ = radical caféoyle, Q₂ = Q₃ = H)
- L'acide 3,4 dicaféoylhexarique (Q₂ = Q₃ = radical caféoyle, Q₁ = Q₄ = H)
- L'acide 3,5 dicaféoylhexarique (Q₂ = Q₄ = radical caféoyle, Q₁ = Q₃ = H)
- L'acide 2,3,4- tricaféoylhexarique (Q₄ = H et Q₁ = Q₂ = Q₃ = radical caféoyle)
- L'acide 2,3,5- tricaféoylhexarique (Q₃ = H et Q₁ = Q₂ = Q₄ = radical caféoyle)
- L'acide léontopodique B (trois quelconques parmi Q₁, Q₂, Q₃ ou Q₄= radical caféoyle et le quatrième radical = H)
- L'acide 3,4,5- tricaféoylhexarique (Q₁ = H et Q₂ = Q₃ = Q₄ = radical caféoyle), c'est à dire l'acide léontopodique B identifié pour la première fois dans les parties aériennes de *Leontopodium alpinum* (Edelweiss), également désigné par « acide léontopodique B1 »
- L'acide léontopodique A (trois quelconques parmi Q₁, Q₂, Q₃ ou Q₄= radical caféoyle et le quatrième radical = groupe CH₃-CH(OH)-CH₂-CO-)

Dans un mode de réalisation particulièrement préféré, les composés pour leur utilisation selon l'invention répondent à la formule (I) dans laquelle trois quelconques des radicaux Q₁, Q₂, Q₃ et Q₄ représentent un groupe caféoyle et le quatrième radical représente un groupe 3-hydroxybutanoyl de formule CH₃-CH(OH)-CH₂-CO-. Plus particulièrement selon ce dernier mode de réalisation, un composé pour son utilisation selon l'invention est l'acide léontopodique A, qui répond à la formule (I) dans laquelle R₁ et R₂ représentent chacun un atome d'hydrogène, trois quelconques des radicaux Q₁, Q₂, Q₃ et Q₄ représentent un groupe caféoyle et le quatrième radical représente un groupe 3-hydroxybutanoyl de formule CH₃-CH(OH)-CH₂-CO-.

Dans un autre mode de réalisation préféré, les composés pour leur utilisation selon l'invention répondent à la formule (I) dans laquelle trois quelconques des radicaux Q₁, Q₂, Q₃ et Q₄ représentent un groupe caféoyle et le quatrième radical représente un atome d'hydrogène. Plus particulièrement selon ce mode de réalisation, un composé pour son utilisation selon l'invention est l'acide léontopodique B, qui répond à la formule (I) dans laquelle R₁ et R₂ représentent chacun un atome d'hydrogène, trois quelconques des radicaux Q₁, Q₂, Q₃ et Q₄ représentent un groupe caféoyle et le quatrième radical représente un atome d'hydrogène.

Selon un aspect encore plus particulier, les composés pour leur utilisation selon l'invention répondent à la formule (I) dans laquelle les radicaux Q₂, Q₃ et Q₄ représentent un groupe caféoyle et le radical Q₁ représente un atome d'hydrogène. Plus particulièrement selon ce mode de réalisation, un composé pour son utilisation selon l'invention est l'acide léontopodique B1, qui répond à la formule (I) dans laquelle R₁ et R₂ représentent chacun un atome d'hydrogène, les radicaux Q₂, Q₃ et Q₄ représentent un groupe caféoyle et le radical Q₁ représente un atome d'hydrogène.

Dans la présente invention, on désigne par « pharmaceutiquement ou nutraceutiquement acceptable » tout ingrédient qui est utile dans la préparation d'une composition pharmaceutique ou nutraceutique, qui est généralement sûr, non toxique et ni biologiquement ni autrement non souhaitable et qui est acceptable pour une utilisation vétérinaire ou chez l'Homme.

On désigne par « sels pharmaceutiquement ou nutraceutiquement acceptables » d'un composé, des sels qui sont pharmaceutiquement ou nutraceutiquement acceptables, comme défini ci-dessus, et qui possèdent l'activité pharmacologique souhaitée du composé parent. De tels sels comprennent :
(1) les hydrates et les solvates,
(2) les sels d'addition d'acide pharmaceutiquement ou nutraceutiquement acceptables formés avec des acides inorganiques pharmaceutiquement ou nutraceutiquement acceptables tels que l'acide chlorhydrique, l'acide bromhydrique, l'acide sulfurique, l'acide nitrique, l'acide phosphorique et similaires ; ou formés avec des acides organiques pharmaceutiquement ou nutraceutiquement acceptables tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires, ou
(3) les sels d'addition de base pharmaceutiquement ou nutraceutiquement acceptables formés lorsqu'un proton acide présent dans le composé parent est soit remplacé par un ion métallique, par exemple un ion de métal alcalin, un ion de métal alcalino-terreux ou un ion d'aluminium ; soit coordonné avec une base organique ou inorganique pharmaceutiquement ou nutraceutiquement acceptable. Les bases organiques acceptables comprennent la diéthanolamine, l'éthanolamine, N-méthylglucamine, la triéthanolamine, la trométhamine et similaires. Les bases inorganiques acceptables comprennent l'hydroxyde d'aluminium, l'hydroxyde de calcium, l'hydroxyde de potassium, le carbonate de sodium et l'hydroxyde de sodium.

Dans le cadre de la présente invention, on entend par « maladie neurodégénérative » une maladie impliquant principalement une détérioration du fonctionnement, et éventuellement la mort, des cellules nerveuses, et en particulier des neurones. Ces maladies induisent des troubles d'ordre cognitivo-comportemental, sensoriel et moteur.

Selon un aspect particulier, ladite maladie neurodégénérative est choisie parmi : la maladie d'Alzheimer, l'ataxie spinocérébelleuse, l'atrophie multisystématisée, la maladie d'Alexander, la maladie d'Alpers, la maladie de Creutzfeldt-Jakob, la maladie de Huntington, la maladie de Parkinson, la maladie de Pick, la myofasciite à macrophages, la paralysie supranucléaire progressive, la sclérose en plaques et la sclérose latérale amyotrophique.

Dans le cadre de la présente invention, une maladie neurodégénérative peut être une maladie neurodégénérative due au stress oxydatif. C'est le cas notamment de la maladie d'Alzheimer.

On entend par « maladie d'Alzheimer » (MA) une maladie affectant principalement les personnes de plus de 65 ans, souffrant de différents symptômes cliniques tels que le déclin progressif de la mémoire, de la pensée, du langage et de la capacité d'apprentissage. Le rôle toxique du peptide β-amyloïde (Aβ) est maintenant passé de fibrilles Aβ insolubles à des plus petits agrégats solubles d'oligomères de Aβ.

Il a été prouvé que des oligomères solubles de Aβ isolé de cortex de patients présentant la maladie d'Alzheimer induisent directement l'hyperphosphorylation de la protéine Tau (T) et la dégénérescence des neurites (Jin M, et al., Soluble amyloid beta-protein dimers isolated from Alzheimer cortex directly induce Tau hyperphosphorylation and neuritic degeneration. Proc Natl Acad Sci USA. 2011 Apr 5;108(14):5819-24). Ainsi, toutes les substances ayant une propriété réductrice de la neurotoxicité du peptide Aβ peuvent être utiles en tant que nouvel agent thérapeutique pour le traitement ou la prévention de la maladie d'Alzheimer.

Selon un autre aspect particulier, les composés pour leur utilisation selon l'invention sont caractérisés en ce que le radical R₁ et le radical R₂ représentent, chacun indépendamment l'un de l'autre, un atome d'hydrogène ou un groupe alkyle en C₂-C_{q} avec q entier naturel compris entre 3 et 18, avantageusement un groupe alkyle en C₂-C₆ et encore plus avantageusement un groupe alkyle en C₂-C₅, éventuellement substitué par un groupe -OH.

Selon un aspect plus particulier, les composés pour leur utilisation selon l'invention sont caractérisés en ce que le radical R₁ et le radical R₂ représentent, chacun indépendamment l'un de l'autre, un atome d'hydrogène ou un groupe choisi parmi les radicaux suivants : éthyle, isopropyle, hydroxy-propyle notamment 2-hydroxy-propyle, hydroxy-pentyle notamment 2-hydroxy-pentyle, (diméthylamino)-éthyle et cinnamyle, et est de préférence un groupe éthyle ou 2-hydroxypentyle.

Selon un aspect encore plus particulier, les composés pour leur utilisation selon l'invention sont caractérisés en ce que :
- le radical R₁ et le radical R₂ représentent, chacun indépendamment l'un de l'autre, un atome d'hydrogène ou un groupe alkyle en C₂-C_{q} avec q entier naturel compris entre 3 et 18, avantageusement un groupe alkyle en C₂-C₆ et encore plus avantageusement un groupe alkyle en C₂-C₅, éventuellement substitué par un groupe -OH, et de préférence un groupe choisi parmi les radicaux suivants : éthyle, isopropyle, hydroxy-propyle notamment 2-hydroxy-propyle, hydroxy-pentyle notamment 2-hydroxy-pentyle, (diméthylamino)-éthyle et cinnamyle,
- trois quelconques des radicaux Q₁, Q₂, Q₃ et Q₄ représentent un groupe caféoyle et le quatrième radical représente un atome d'hydrogène.

Selon un aspect particulièrement préféré, les composés pour leur utilisation selon l'invention sont caractérisés en ce que le radical R₁ et le radical R₂, identiques ou différents, sont choisis parmi les radicaux suivants : éthyle, isopropyle, hydroxy-propyle notamment 2-hydroxy-propyle, hydroxy-pentyle notamment 2-hydroxy-pentyle, (diméthylamino)-éthyle et cinnamyle, et trois quelconques des radicaux Q₁, Q₂, Q₃ et Q₄ représentent un groupe caféoyle et le quatrième radical représente un atome d'hydrogène.

Selon un aspect plus particulièrement préféré, les composés pour leur utilisation selon l'invention sont choisis parmi :
- les composés dans lesquels R₁ et R₂ représentent un groupe éthyle, trois quelconques des radicaux Q₁, Q₂, Q₃ et Q₄ représentent un groupe caféoyle et le quatrième radical représente un atome d'hydrogène ;
- les composés dans lesquels R₁ et R₂ représentent un groupe 2-hydroxy-pentyle, trois quelconques des radicaux Q₁, Q₂, Q₃ et Q₄ représentent un groupe caféoyle et le quatrième radical représente un atome d'hydrogène ;
- les composés dans lesquels R₁ et R₂ représentent un groupe 2-hydroxy-propyle, trois quelconques des radicaux Q₁, Q₂, Q₃ et Q₄ représentent un groupe caféoyle et le quatrième radical représente un atome d'hydrogène ;
- les composés dans lesquels R₁ et R₂ représentent un groupe isopropyle, trois quelconques des radicaux Q₁, Q₂, Q₃ et Q₄ représentent un groupe caféoyle et le quatrième radical représente un atome d'hydrogène ;
- les composés dans lesquels R₁ et R₂ représentent un groupe (diméthylamino)-éthyle, trois quelconques des radicaux Q₁, Q₂, Q₃ et Q₄ représentent un groupe caféoyle et le quatrième radical représente un atome d'hydrogène ; et
- les composés dans lesquels R₁ et R₂ représentent un groupe cinnamyle, trois quelconques des radicaux Q₁, Q₂, Q₃ et Q₄ représentent un groupe caféoyle et le quatrième radical représente un atome d'hydrogène.

En particulier, parmi les composés de formule (I) avantageux pour leur utilisation selon la présente invention, on peut citer les dérivés esters de :
- L'acide 2 caféoylhexarique (Q₁ = radical caféoyle, Q₂ = Q₃ = Q₄ = H)
- L'acide 3 caféoylhexarique (Q₂ = radical caféoyle, Q₁ = Q₃ = Q₄ = H)
- L'acide 4 caféoylhexarique (Q₃ = radical caféoyle, Q₁ = Q₂ = Q₃ = H)
- L'acide 5 caféoylhexarique (Q₄ = radical caféoyle, Q₁ = Q₂ = Q₃ = H)
- L'acide 2,3 dicaféoylhexarique (Q₁ = Q₂ = radical caféoyle, Q₃ = Q₄ = H)
- L'acide 2,4 dicaféoylhexarique (Q₁ = Q₃ = radical caféoyle, Q₂ = Q₄ = H)
- L'acide 2,5 dicaféoylhexarique (Q₁ = Q₄ = radical caféoyle, Q₂ = Q₃ = H)
- L'acide 3,4 dicaféoylhexarique (Q₂ = Q₃ = radical caféoyle, Q₁ = Q₄ = H)
- L'acide 3,5 dicaféoylhexarique (Q₂ = Q₄ = radical caféoyle, Q₁ = Q₃ = H)
- L'acide 2,3,4- tricaféoylhexarique (Q₄ = H et Q₁ = Q₂ = Q₃ = radical caféoyle)
- L'acide 2,3,5- tricaféoylhexarique (Q₃ = H et Q₁ = Q₂ = Q₄ = radical caféoyle)
- L'acide léontopodique B (trois quelconques parmi Q₁, Q₂, Q₃ ou Q₄= radical caféoyle et le quatrième radical = H)
- L'acide 3,4,5- tricaféoylhexarique (Q₁ = H et Q₂ = Q₃ = Q₄ = radical caféoyle), c'est à dire l'acide léontopodique B identifié pour la première fois dans les parties aériennes de *Leontopodium alpinum* (Edelweiss), également désigné par « acide léontopodique B1 »
- L'acide léontopodique A (trois quelconques parmi Q₁, Q₂, Q₃ ou Q₄= radical caféoyle et le quatrième radical = groupe CH₃-CH(OH)-CH₂-CO-)

Parmi les composés avantageux pour leur utilisation selon la présente invention, on peut citer les dérivés esters suivants :
- L'éthyle diester de l'acide léontopodique B, et notamment l'éthyle diester de l'acide léontopodique B1,
- Le 2-hydroxy-pentyle diester de l'acide léontopodique B, et notamment le 2-hydroxy-pentyle diester de l'acide léontopodique B1.

Selon un autre aspect plus particulier, l'invention a pour objet des composés caractérisés en ce que Q₂, Q₃ et Q₄ représentent chacun un radical caféoyle, Q₁ représente un atome d'hydrogène, et en ce que le radical R₁ et le radical R₂ représentent, chacun indépendamment l'un de l'autre, un groupe alkyle en C₂- C₆, éventuellement substitué par un groupe -OH, notamment R₁ et R₂ représentent un groupe 2-hydroxy-pentyle, ou notamment R₁ et R₂ représentent un groupe 2-hydroxy-propyle, leurs stéréoisomères et leurs sels pharmaceutiquement ou nutraceutiquement acceptables, pour leur utilisation pour la prévention et/ou le traitement de la maladie d'Alzheimer.

De manière particulièrement préférée, l'invention a pour objet un composé choisi parmi : l'acide léontopodique B, l'éthyle ester de l'acide léontopodique B et le 2-hydroxy-pentyle ester de l'acide léontopodique B pour son utilisation pour la prévention et/ou le traitement de la maladie d'Alzheimer.

Selon un aspect plus particulier, l'invention a pour objet l'acide léontopodique B, répondant à la formule (I) dans laquelle trois quelconques des radicaux Q₁, Q₂, Q₃ et Q₄ représentent chacun un radical caféoyle, le quatrième radical représente un atome d'hydrogène, et en ce que R₁ et R₂ représentent un atome d'hydrogène, pour son utilisation pour la prévention et/ou le traitement de la maladie d'Alzheimer.

Selon un autre aspect plus particulier, l'invention a pour objet l'éthyle ester de l'acide léontopodique B, répondant à la formule (I) dans laquelle trois quelconques des radicaux Q₁, Q₂, Q₃ et Q₄ représentent chacun un radical caféoyle, le quatrième radical représente un atome d'hydrogène, et en ce que R₁ et R₂ représentent un groupe éthyle, pour leur utilisation pour la prévention et/ou le traitement de la maladie d'Alzheimer.

Selon un autre aspect plus particulier, l'invention a pour objet l'hydroxy-pentyle ester de l'acide léontopodique B, répondant à la formule (I) dans laquelle trois quelconques des radicaux Q₁, Q₂, Q₃ et Q₄ représentent chacun un radical caféoyle, le quatrième radical représente un atome d'hydrogène, et en ce que R₁ et R₂ représentent un groupe hydroxy-pentyle, notamment un groupe 2-hydroxy-pentyle, pour leur utilisation pour la prévention et/ou le traitement de la maladie d'Alzheimer.

Selon un deuxième aspect, l'invention a pour objet des composés de formule générale (I) 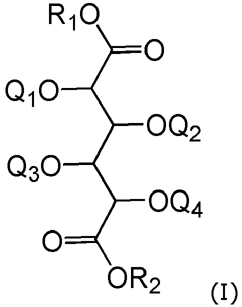 dans laquelle
R₁ et R₂ sont identiques ou différents et représentent, chacun indépendamment l'un de l'autre, un atome d'hydrogène ou un groupe alkyle en C₁-Cₚ linéaire ou ramifié, cyclique ou cyclique ramifié, saturé ou insaturé, pouvant être substitué par un ou plusieurs substituants choisis dans le groupe comprenant : -OR₃, - NR₃R₄, -NO₂, -COR₃, -COOR₃, -CF₃, -CR₃F₂, -CR₃R₄F, =O, -CN, halogène, -SR₃, -SO₂R₃, aryle, avec
   - p est un entier naturel compris entre 2 et 18,
   - R₃ et R₄ sont identiques ou différents et représentent chacun indépendamment l'un de l'autre,
      - soit un groupe choisi dans le groupe comprenant : -H, -OH, - NH₂, -NO₂, -COOH, -CF₃, -CHF₂, -CH₂F, =O, -CN, halogène, - SH et - SO₂H,
      - soit un groupe alkyle en C₁-C₆ linéaire ou ramifié, cyclique ou cyclique ramifié, saturé ou insaturé, pouvant être substitué par un ou plusieurs substituants choisis dans le groupe comprenant : -H, -OH, -NH₂, -NO₂, -COOH, -CF₃, -CHF₂, -CH₂F, =O, -CN, halogène, -SH et -SO₂H,
         sous réserve qu'au moins un des radicaux R₁ ou R₂ ne soit pas un atome d'hydrogène,
         et
Q₁, Q₂, Q₃ et Q₄ représentent chacun, indépendamment l'un de l'autre, un radical choisi dans le groupe comprenant : un atome d'hydrogène, un groupe caféoyle et un groupe CH3- CH(OH)- CH₂CO-, sous réserve qu'au moins un de ces radicaux soit un groupe caféoyle,
à l'exception des composés suivants :
   - 1-O-méthyle ester de 2,3,4-tricaféoyle d'acide altrarique
   - 1-O-méthyle ester de 2,3-dicaféoyle d'acide altrarique
   leurs stéréoisomères et leurs sels pharmaceutiquement ou nutraceutiquement acceptables.

Selon un mode avantageux de réalisation de cet aspect de l'invention, dans les composés de formule (I) trois quelconques des radicaux Q₁, Q₂, Q₃ et Q₄ représentent un groupe caféoyle, et le quatrième radical représente un atome d'hydrogène.

Selon un mode plus avantageux de réalisation de cet aspect de l'invention, dans les composés de formule (I) Q₂, Q₃ et Q₄ représentent un groupe caféoyle.

Selon un mode encore plus avantageux de réalisation de cet aspect de l'invention, dans les composés de formule (I) Q₂, Q₃ et Q₄ représentent un groupe caféoyle et Q₁ représente un atome d'hydrogène.

Selon un autre mode avantageux de réalisation de cet aspect de l'invention, dans les composés de formule (I) Q₂, Q₃ et Q₄ représentent un groupe caféoyle et Q₁ représente un groupe CH₃-CH(OH)-CH₂CO-.

Selon un autre aspect particulier, un composé selon l'invention est caractérisé en ce que R₁ et R₂ représentent, chacun indépendamment l'un de l'autre, un atome d'hydrogène ou un groupe alkyle en C₁-C₁₅, avantageusement un groupe alkyle en C₁-C₁₂, plus avantageusement un groupe alkyle en C₂-C₆, et encore plus avantageusement un groupe alkyle en C₂, C₅ ou C₆, sous réserve qu'au moins un des radicaux R₁ ou R₂ ne soit pas un atome d'hydrogène.

Selon un aspect plus particulier, un composé selon l'invention est caractérisé en ce que trois quelconques des radicaux Q₁, Q₂, Q₃ et Q₄ représentent un groupe caféoyle, et le quatrième radical représente un atome d'hydrogène, et R₁ et R₂ représentent, chacun indépendamment l'un de l'autre, un groupe alkyle en C₂- C₆ substitué par un groupe -OH, notamment un groupe 2-hydroxy-pentyle ou un groupe 2-hydroxy-propyle, sous réserve qu'au moins un des radicaux R₁ ou R₂ ne soit pas un atome d'hydrogène.

Parmi les composés avantageux selon la présente invention, on peut citer les dérivés esters suivants :
- L'éthyle diester de l'acide léontopodique B, et notamment l'éthyle diester de l'acide léontopodique B1,
- Le 2-hydroxy-pentyle diester de l'acide léontopodique B, et notamment le 2-hydroxy-pentyle diester de l'acide léontopodique B1.

Les composés de formule générale (I) selon l'invention sont préparés par la mise en œuvre d'un procédé d'estérification d'une fonction acide avec un alcool approprié selon les méthodes connues de l'homme du métier. Ledit procédé comprend de préférence une étape durant laquelle un acide glucarique ou un acide altrarique polysubstitué réagit avec un alcool de formule R-OH.

Selon ce procédé, ledit acide glucarique polysubstitué est l'acide léontopodique, et notamment l'acide léontopodique B.

Les inventeurs ont développé un procédé de préparation des dérivés ester de PCH en une étape par hémisynthèse à partir d'un acide glucarique ou d'un acide altrarique particulier permettant l'obtention de dérivés esters d'un acide glucarique ou d'un acide altrarique sous la forme d'un seul régioisomère. Ce procédé de préparation utilisé correspond à la réaction d'estérification décrite dans l'article de Hanessian et al. (2001) (Stephen Hanessian, Hoan K Huynh, Gurijala V Reddy, Rudolph O Duthaler, Andreas Katopodis, Markus B Streiff, Willy Kinzy, Reinhold Oehrlein. Synthesis of Gal determinant epitopes, their glycomimetic variants, and trimeric clusters-relevance to tumor associated antigens and to discordant xenografts. Tetrahedron, 16 April 2001, Volume 57, Issue 16, Pages 3281-3290).

L'alcool est choisi parmi les alcools de formule R-OH, dans laquelle R représente un groupe alkyle en C₁-Cₚ linéaire ou ramifié, cyclique ou cyclique ramifié, saturé ou insaturé, où p est un entier naturel compris entre 2 et 18, ledit groupe pouvant être substitué par un ou plusieurs substituants choisis dans le groupe comprenant : -OR₁, -NR₁R₂, -NO₂, -COR₁, -COOR₁, -CF₃, -CR₁F₂, -CR₁R₂F, =O, - CN, halogène, -SR₁, -SO₂R₁, aryle, où R₁ et R₂ sont identiques ou différents et représentent, chacun indépendamment l'un de l'autre, soit un groupe alkyle en C₁-C₆ linéaire ou ramifié, cyclique ou cyclique ramifié, saturé ou insaturé, pouvant être substitué par un ou plusieurs substituants choisis dans le groupe comprenant : -H, -OH, -NH₂, -NO₂, - COOH, -CF₃, -CHF₂, -CH₂F, =O, -CN halogène, -SH, -SO₂H, soit un substituant choisi dans le groupe comprenant : - H, -OH, -NH₂, -NO₂, -COOH, -CF₃, -CHF₂, -CH₂F, =O, -CN, halogène, -SH, - SO₂H.

Plus particulièrement, l'alcool est choisi parmi les alcools de formule R-OH, dans laquelle R représente un groupe alkyle en C₂-C₁₅, avantageusement un groupe alkyle en C₂-C₁₂, plus avantageusement un groupe alkyle en C₂-C₆, et encore plus avantageusement un groupe alkyle en C₂, C₅ ou C₆.

Encore plus particulièrement, l'alcool est choisi parmi les alcools de formule R- OH, dans laquelle R est un groupe alkyle en C₂-C₆ substitué par un groupe -OH, et notamment R est un groupe 2-hydroxy-pentyle ou un groupe 2- hydroxy-propyle.

De manière encore plus préférée, l'alcool est choisi parmi : l'éthanol, le 2-hydroxy-pentan-1-ol et le 2-hydroxy-propan-1-ol.

Dans un mode de réalisation de l'invention, l'étape du procédé durant laquelle un acide glucarique ou un acide altrarique polysubstitué, avantageusement l'acide léontopodique, et plus avantageusement l'acide léontopodique B, réagit avec un composé alcool de formule R-OH est éventuellement précédée d'une étape d'activation du groupe carboxyle d'un acide glucarique ou un acide altrarique polysubstitué, avantageusement l'acide léontopodique, et plus avantageusement l'acide léontopodique B, notamment par un agent d'activation des groupes carboxyles, tel que le 1-(3-dimethylaminopropyl)-3-ethylcarbodiimide en combinaison avec l'hydrate 1-hydroxybenzotriazole.

Par « agent d'activation du groupe carboxyle », on entend selon la présente invention tout réactif ou combinaison de réactifs permettant d'activer la fonction acide carboxylique pour permettre son couplage avec un nucléophile dans des conditions réactionnelles douces. A titre d'exemple et de façon non exhaustive, on peut citer les agents d'activation de la famille des carbodiimides comme le dicyclohexylcarbodiimide (DCC), le diisopropylcarbodiimide (DIC), le N-éthyle-N(3-dimethylaminopropyl)carbodiimide (EDCI) seul ou en combinaison avec des alcools permettant la formation transitoire d'esters activés comme, par exemple, le 1-hydroxybenzotriazole (HOBT), le 1-hydroxy-7-azabenzotriazole (HOAt), le 1-hydroxysuccinimide (HOSu) ou encore l'éthyle (hydroxyimino)cyanoacetate. L'agent d'activation utilisable peut également faire partie de la famille des sels de phosphoniums, d'uronium et/ou de guanidinium. A titre d'exemple, on peut citer le benzotriazol-1-yloxy)tris(dimethylamino)phosphonium hexafluorophosphate (BOP), le benzotriazol-1-yl-oxytripyrrolidinophosphonium hexafluorophosphate (PyBOP), le (1-Cyano-2-ethoxy-2-oxoethylidenaminooxy)dimethylamino-morpholino-carbenium hexafluorophosphate (COMU), N,N,N',N'-Tetramethyl-O-(1H-benzotriazol-1-yl)uronium hexafluorophosphate (HBTU), (O-(6-Chloro-1-hydrocibenzotriazol-1-yl)-1,1,3,3-tetramethyluronium tetrafluoroborate) (TCTU), (2-(7-Aza-1H-benzotriazole-1-yl)-1,1,3,3-tetramethyluronium hexafluorophosphate) (HATU).

Plus particulièrement, l'agent d'activation des groupes carboxyles est choisi parmi le diisopropylcarbodiimide (DIC) et le 1-hydroxybenzotriazole (HOBT). Les procédés de préparation des composés selon l'invention peuvent éventuellement comprendre des étapes supplémentaires de protection et/ou déprotection afin d'éviter des réactions secondaires bien connues de l'homme du métier, ou afin d'éviter la formation de plusieurs régioisomères des composés selon l'invention.

Les composés obtenus par les procédés selon l'invention peuvent en outre être purifiés par des méthodes connues de l'homme du métier. On peut citer, par exemple, les méthodes de purification par cristallisation, par chromatographie ou par extraction.

Dans un troisième aspect, l'invention concerne donc des composés de formule générale (I) : 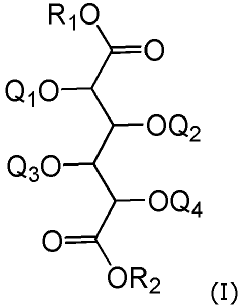 dans laquelle
R₁ et R₂ sont identiques ou différents et représentent, chacun indépendamment l'un de l'autre, un atome d'hydrogène ou un groupe alkyle en C₁-Cₚ linéaire ou ramifié, cyclique ou cyclique ramifié, saturé ou insaturé, pouvant être substitué par un ou plusieurs substituants choisis dans le groupe comprenant : -OR₃, - NR₃R₄, -NO₂, -COR₃, -COOR₃, -CF₃, -CR₃F₂, -CR₃R₄F, =O, -CN, halogène, -SR3, -SO₂R₃, aryle, avec
   - p est un entier naturel compris entre 2 et 18,
   - R₃ et R₄ sont identiques ou différents et représentent chacun indépendamment l'un de l'autre,
   - soit un groupe choisi dans le groupe comprenant : -H, -OH, - NH₂, -NO₂, -COOH, -CF₃, -CHF₂, -CH₂F, =O, -CN, halogène, - SH et - SO₂H,
   - soit un groupe alkyle en C₁-C₆ linéaire ou ramifié, cyclique ou cyclique ramifié, saturé ou insaturé, pouvant être substitué par un ou plusieurs substituants choisis dans le groupe comprenant : -H, -OH, -NH₂, -NO₂, -COOH, -CF₃, -CHF₂, -CH₂F, =O, -CN, halogène, -SH et -SO₂H,
sous réserve qu'au moins un des radicaux R₁ ou R₂ ne soit pas un atome d'hydrogène,
   et
Q₁, Q₂, Q₃ et Q₄ représentent chacun, indépendamment l'un de l'autre, un radical choisi dans le groupe comprenant : un atome d'hydrogène, un groupe caféoyle et un groupe CH3- CH(OH)- CH₂CO-, sous réserve qu'au moins un de ces radicaux soit un groupe caféoyle,
leurs stéréoisomères et leurs sels pharmaceutiquement ou nutraceutiquement acceptables, pour leur utilisation en tant que médicament.

Selon un aspect plus particulier, la présente invention concerne des composés de formule générale (I) : 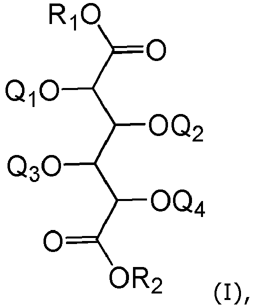 dans laquelle R₁, R₂, Q₁, Q₂, Q₃ et Q₄ sont tels que définis ci-dessus, ou leurs sels et stéréoisomères pharmaceutiquement ou nutraceutiquement acceptables, pour leur utilisation en tant que médicament pour la prévention et/ou le traitement d'une maladie neurodégénérative, en particulier la maladie d'Alzheimer.

Plus particulièrement, la présente invention concerne l'acide léontopodique B, l'acide léontopodique B1, l'éthyle diester de l'acide léontopodique B, le 2-hydroxy-pentyle diester de l'acide léontopodique B et le 2-hydroxy-propyle diester de l'acide léontopodique B pour leur utilisation en tant que médicament, notamment pour la prévention et/ou le traitement d'une maladie neurodégénérative, en particulier la maladie d'Alzheimer.

Il est aussi divulgué une méthode de prévention et/ou de traitement d'une maladie neurodégénérative, notamment d'une des maladies citées ci-dessus, comprenant l'administration d'une quantité thérapeutiquement efficace d'au moins un composé de formule (I) selon l'invention à un patient en ayant besoin. Plus particulièrement, il est divulgué une méthode de prévention et/ou de traitement de la maladie d'Alzheimer, comprenant l'administration d'une quantité thérapeutiquement efficace d'au moins un composé de formule (I) selon l'invention à un patient en ayant besoin, et comprenant notamment l'administration d'un composé choisi parmi : l'acide léontopodique B, l'acide léontopodique B1, l'éthyle diester de l'acide léontopodique B et le 2-hydroxy-pentyle diester de l'acide léontopodique B.

Selon un quatrième aspect, la présente invention est également relative à une composition pharmaceutique comprenant en tant qu'agent actif au moins un composé ou un mélange de composés de formule générale (I) dans laquelle R₁, R₂, Q₁, Q₂, Q₃ et Q₄ sont tels que définis ci-dessus, ou ses sels et stéréoisomères pharmaceutiquement acceptables, et avantageusement un excipient pharmaceutiquement acceptable.

Les modes d'administration, les posologies et les formes galéniques optimales d'une composition pharmaceutique selon l'invention peuvent être déterminés selon les critères généralement pris en compte dans l'établissement d'un traitement pharmaceutique adapté à un sujet comme par exemple l'âge ou le poids corporel du patient, la gravité de son état général, la tolérance au traitement, les effets secondaires constatés, le type de peau. En fonction du type d'administration souhaitée, la composition pharmaceutique selon l'invention peut en outre comprendre au moins un excipient pharmaceutiquement acceptable. La composition pharmaceutique selon la présente invention peut en outre comprendre au moins un adjuvant pharmaceutiquement connu de l'homme du métier, choisi parmi les épaississants, les conservateurs, les parfums, les colorants, des filtres chimiques ou minéraux, les agents hydratants, les eaux thermales, etc.

Avantageusement, la composition pharmaceutique comprend au moins un composé de formule générale (I) selon l'invention en une quantité comprise entre 0,01 et 10%, en particulier entre 0,05 et 5%, plus particulièrement entre 0,1 et 2%, en poids par rapport au poids total de la composition.

La composition pharmaceutique est particulièrement adaptée pour une administration par voie orale, nasale, transdermique, parentérale, topique, rectale, et mucosale. Elle peut se présenter sous forme sèche, telle que par exemple : capsule molle, gélule, comprimé, lyophilisat, poudre, granule, ou patch, ou sous forme liquide, telle que : solution, suspension, spray, crème ou gel.

L'excipient pharmaceutiquement acceptable est connu de l'Homme du Métier et est choisi selon le mode d'administration de la composition pharmaceutique. A titre d'exemple, l'excipient pharmaceutiquement acceptable peut être choisi dans le groupe constitué par les agents diluants, liants, désintégrants, les colorants, les lubrifiants, les agents solubilisants, les agents promoteurs d'absorption, les filmogènes, les agents gélifiants, et leurs mélanges.

La composition pharmaceutique selon l'invention peut en outre comprendre au moins un composé choisi dans le groupe constitué par les émollients, les actifs hydratants, les activateurs de la synthèse de kératine, les kératorégulateurs, les kératolytiques, les agents restructurant de la barrière cutanée (activateurs de la synthèse des lipides cutanés, agonistes PPARs ou Peroxysome Proliferator Activated Receptor), les activateurs de la différenciation des kératinocytes (rétinoïdes, calcidone®, le calcium), les antibiotiques, les agents anti-bactériens, les composés antifongiques, les agents anti-viraux, les sébo-régulateurs, les immunomodulateurs, tels que le tacrolimus, le pimécrolimus, les oxazolines, les conservateurs, les agents anti-irritants, les agents apaisants, des filtres et écrans solaires, les agents anti-oxydants, les facteurs de croissance, les agents cicatrisants ou les molécules eutrophiques, les médicaments et les agents anti-Alzheimer.

La présente invention concerne également une composition pharmaceutique selon l'invention pour son utilisation dans la prévention et/ou le traitement de maladies neurodégénératives, en particulier la maladie d'Alzheimer.

Il est également divulgué une méthode de prévention et/ou de traitement, notamment de maladies neurodégénératives, notamment d'une des maladies citées ci-dessus, et particulièrement la maladie d'Alzheimer, comprenant l'administration d'une quantité thérapeutiquement efficace d'une composition pharmaceutique selon l'invention à un patient en ayant besoin.

La présente invention concerne également une composition nutraceutique comprenant un composé de formule (I) selon l'invention, et avantageusement un excipient nutraceutiquement acceptable, pour améliorer les fonctions cognitives chez un patient atteint d'une maladie neurodégénérative, et particulièrement la maladie d'Alzheimer. L'excipient nutraceutiquement acceptable est connu de l'Homme du Métier et est choisi parmi les excipients conformes aux règlementations internationales applicables aux additifs alimentaires.

Les exemples qui suivent et les figures 1, 2, 3 et 4 visent à illustrer la présente invention, sans toutefois en limiter la portée.

### EXEMPLES

### Exemple 1 : Effet de l'acide léontopodique B1 sur la survie de neurones corticaux primaires blessés par le peptide Aβ (20 µg, 24 heures) et sur un réseau de neurites de neurones corticaux primaires blessés par le peptide Aβ (20 µg, 24 heures).

Le but de cette étude est d'évaluer les effets de l'acide léontopodique B1 sur des neurones corticaux primaires de rat, intoxiqués par le peptide Aβ selon le modèle in vitro de la maladie d'Alzheimer décrit dans Callizot *et al.,* 2013 (Callizot N. et al., Operational dissection of β-amyloid cytopathic effects on cultured neurons. J Neurosci Res. 2013, 91: 706-16). La survie des neurones et des astrocytes ainsi que l'intégrité du réseau de neurites sont évaluées.

### 1.1. Matériels et méthodes

### Acide léontopodique B1

Fournisseur : EXTRASYNTHESE (Code d'identification # 6026 S ; CAS [933063-22-0] ; Formule C33H28O17 ; Poids moléculaire 696.58 ; Degrés de pureté (selon le chromatogramme HPLC) ≥98% ; extrait de *Leontopodium alpinum* (Edelweiss)).
Quatre concentrations sont testées : 10 nM, 100 nM, 1 µM et 10 µM
Le véhicule utilisé est le milieu de culture (voir la section ci-dessous) contenant 0,1% de DMSO (Pan Biotech, Batch: H130813).

### Culture cellulaire de neurones corticaux

Des neurones corticaux de rat sont cultivés comme décrit dans Singer *et al.,* 1999 (Singer C. et al., Mitogen-activated protein kinase pathway mediates estrogen neuroprotection after glutamate toxicity in primary cortical neurons. J Neurosci. 1999, 19: 2455-2463) et Callizot *et al.* (2013).

En résumé, des rats femelles gravides sont tuées par dislocation cervicale après 15 jours de gestation. Les fœtus sont collectés et immédiatement placés dans un milieu glacé L15 Leibovitz (Pan Biotech, Batch: 8810315) additionné de 2% pénicilline (10,000 U/mL) et d'une solution de streptomycine (10 mg/mL) (PS; Pan Biotech, batch: 1451013) et 1% de albumine de sérum de bovin (BSA; Pan Biotech, batch: K180713). Le cortex est traité pendant 20 min à 37°C avec une solution de trypsine- EDTA (Pan Biotech, Batch: 3330914) à une concentration finale de 0,05% trypsine et 0,02% EDTA. La dissociation est stoppée par l'ajout du milieu DMEM (Dulbecco's modified Eagle's medium) avec 4,5 g/L de glucose (Pan Biotech, Batch: 8530315), contenant de la DNase I grade II (concentration finale 0.5 mg/mL; Pan Biotech, Batch: H140508) et 10% sérum de veau fœtal (FCS; Invitrogen, Batch: 41Q1613K). Les cellules sont mécaniquement dissociées par trois passages forcés à travers des cônes de pipette de 10 mL. Les cellules sont ensuite centrifugées à 515g pendant 10 min à 4°C. Le surnageant est jeté, et le culot est suspendu dans un milieu de culture défini consistant en un milieu Neurobasal (Invitrogen, batch: 1715722) avec une solution à 2% de supplément B27 (Invitrogen, lot: 1709534), 2 mmol/L de L-glutamine (Pan Biotech, lot: 6620314), 2% de solution de PS, et 10 ng/mL de facteur neurotrophique dérivé du cerveau (BDNF; Pan Biotech, Batch: H140108). Les cellules viables sont comptées avec un cytomètre Neubauer, en utilisant le test d'exclusion du bleu trypan. Les cellules sont ensemencées à une densité de 30,000 par puits d'une plaque de 96 puits préenduits de poly-L-lysine (Biocoat, Batch: 21614030) et sont cultivées à 37°C dans un incubateur contenant 95% d'air et 5% de CO₂. Le milieu est changé tous les jours. Après 11 jours de culture, les neurones corticaux sont intoxiqués par une solution de peptides Aβ (voir ci-dessous).

### Préparation du peptide Aβ 1-42 et exposition des neurones corticaux audit peptide en présence d'acide léontopodique B1

La préparation du peptide Aβ 1-42 est réalisée selon la procédure décrite dans Callizot *et al.* (2013). En résumé, le peptide Aβ 1-42 (Bachem, Batch: 1014012) est dissous dans le milieu de culture défini mentionné ci-dessus, dépourvu de sérum, à une concentration initiale de 40 µmoles/L. Cette solution est doucement agitée pendant 3 jours à 37°C dans le noir et immédiatement utilisée après avoir été diluée dans le milieu de culture à la concentration testée (20 µM).

Au onzième jour de culture, l'acide léontopodique B1 testé aux 4 différentes concentrations est dissous dans le milieu de culture et est ensuite préincubé avec les neurones corticaux primaires pendant 1 heure avant l'application du peptide Aβ 1-42. La solution de peptide Aβ 1-42 est ajoutée à une concentration finale de 20 µM diluée dans le milieu de culture en présence de l'acide léontopodique B1 et le tout est laissé pendant 24 heures.

### Evaluation de la survie des neurones et des astrocytes ainsi que de l'intégrité du réseau de neurites

24 heures après intoxication par le peptide Aβ 1-42, le surnageant de la culture cellulaire a été enlevé et les cultures de neurones corticaux ont été fixées par une solution froide d'éthanol (95%, Sigma, Batch: SZBD3080V) et d'acide acétique (5%, Sigma, Batch: SZBD1760V) pendant 5 min à -20°C. Après perméabilisation avec 0,1% de saponine (Sigma, Batch: BCBJ8417V), les cellules sont incubées pendant 2 heures :
a) avec un anticorps de souris monoclonal anti microtubule-associated-protein 2 (MAP-2, Callizot N. et al., A new long term in vitro model of myelination. Experimental Cell Research, October 2011. Volume 317, Issue 16, Pages 2374-2383), Sigma, batch: 063M4802) dilué à 1/400 dans du PBS (PAN, Batch: 1870415) contenant 1% de sérum de veau fœtal (FCS) et 0,1% de saponine (cet anticorps spécifique des corps cellulaires et des neurites permet l'étude de la mort des cellules neuronales ainsi que l'effet de l'acide léontopodique B1 sur la croissance des neurites) pendant 2 heures à température ambiante,
b) avec un anticorps polyclonal de lapin anti-protéine acide fibrillaire gliale (GFAP, Sigma, Batch: 083M4830) à une dilution de 1/400 dans du PBS contenant 1% FCS, 0.1 % saponine, pendant 2 heures à température ambiante. Cet anticorps est spécifique des astrocytes.

Ces anticorps sont révélés par un IgG de chèvre anti-souris marqué par un Alexa Fluor 488 (Molecular Probe, Batch: 1664729) et un IgG de chèvre anti-lapin marqué par un Alexa Fluor 568 (Molecular Probe, Batch: 1386541) à une dilution de 1/400 dans du PBS contenant 1% FCS, 0,1% saponine, pendant 1 heure à température ambiante.

L'acide léontopodique B1 est testé pour une culture. Pour chaque concentration d'acide léontopodique B1 et les contrôles, 6 puits sont évalués, 30 photos par plaque sont prises en utilisant ImageXpress (Molecular Devices) avec un grossissement 20x, pour évaluer le réseau de neurites, le nombre de neurones et le nombre d'astrocytes. L'analyse des photos est réalisée en utilisant Custom Module Editor (Molecular Devices). Les résultats sont présentés en termes de nombre de cellules positives ou de réseau de neurite marqués par marqueur donné (MAP-2, GFAP).

Chaque ligne de la plaque est organisée de la façon suivante :
- Pas de traitement (contrôle négatif) / 0.1% DMSO
- Peptide Aβ 1-42 20 µM / 0,1% DMSO
- Peptide Aβ 1-42 20 µM / acide léontopodique B1 10 nM / 0,1% DMSO
- Peptide Aβ 1-42 20 µM / acide léontopodique B1 100 nM / 0,1% DMSO
- Peptide Aβ 1-42 20 µM / acide léontopodique B1 1 µM / 0,1% DMSO
- Peptide Aβ 1-42 20 µM / acide léontopodique B1 10 µM / 0,1% DMSO Chaque ligne est répétée 6 fois.

### Analyse statistique

Toutes les données sont exprimées en pourcentage du contrôle des conditions (contrôle = 100 %). Toutes les valeurs sont exprimées en moyenne +/- l'erreur standard de la moyenne (Standard Error of the Mean SEM) (s.e.mean) de n = 6 puits par condition. Les analyses statistiques réalisées pour les différentes conditions sont des analyses de variances ou ANOVA suivie par le test PLSD de Fischer ou le test de Dunnett en utilisant le logiciel GraphPad Prism (* p < 0.05 vs condition contrôle).

### 1.2. Résultats

La Figure 1 représente l'effet de l'acide léontopodique B1 sur la survie des neurones corticaux primaires intoxiqués pendant 24 heures par le peptide Aβ 1-42 ajouté à une concentration finale de 20 µM. Les données sont exprimées en pourcentage du contrôle (100% = pas de traitement) comme moyenne +/l'erreur standard de la moyenne (Standard Error of the Mean SEM) (s.e.mean) de n = 6 puits par condition (ANOVA suivie par le test PLSD de Fischer en utilisant le logiciel GraphPad Prism. (* p < 0.05 vs condition contrôle).

La Figure 2 représente l'effet de l'acide léontopodique B1 sur le réseau de neurites intoxiqués pendant 24 heures par le peptide Aβ 1-42 ajouté à une concentration finale de 20 µM. Ces données permettent de vérifier l'état de santé des neurones corticaux, car le stress engendré par la pathologie Alzheimer par le biais du peptide Aβ 1-42 affecte le nombre de connexions neuronales (neurites). Les données sont exprimées en pourcentage du contrôle (100% = pas de traitement) comme moyenne +/- l'erreur standard de la moyenne (Standard Error of the Mean SEM) (s.e.mean) de n = 6 puits par condition (ANOVA suivie par le test PLSD de Fischer en utilisant le logiciel GraphPad Prism. (* p < 0.05 vs condition contrôle).

La Figure 3 représente l'effet de l'acide léontopodique B1 sur la survie des astrocytes intoxiqués pendant 24 heures par le peptide Aβ 1-42 ajouté à une concentration finale de 20 µM. Les données sont exprimées en pourcentage du contrôle (100% = pas de traitement) comme moyenne +/- l'erreur standard de la moyenne (Standard Error of the Mean SEM) (s.e.mean) de n = 6 puits par condition (ANOVA suivie par le test PLSD de Fischer en utilisant le logiciel GraphPad Prism. (* p < 0.05 vs condition contrôle).

Le témoin contrôle n'a pas été traité avec le peptide Aβ 1-42. Seul le véhicule utilisé pour l'acide léontopodique B1 (le milieu de culture contenant 0,1% de DMSO) a été ajouté à la culture cellulaire de neurones corticaux. On observe 100% de survie des neurones dans cette condition.

L'ajout du peptide Aβ 1-42 à une concentration finale de 20 µM (20 µM - 24h), induit au bout de 24 heures une mort significative des neurones (Figure 1), des astrocytes (Figure 3) ainsi qu'une perte significative du réseau de neurites (Figure 2) comme précédemment indiqué dans la littérature (Callizot *et al.,* 2013).

L'acide léontopodique B1 pre-incubé 1 heure avec les cellules neuronales en culture avant l'ajout du peptide Aβ 1-42 montre un effet significatif et important sur la survie des neurones aux 4 concentrations (10 nM, 100 nM, 1 µM, 10 µM) (Figure 1). À 100 nM et 1 µM, aucune mort neuronale n'a été observée. L'effet protecteur suit une courbe en forme de cloche. L'acide léontopodique B1 pre-incubé 1 heure avec les cellules neuronales en culture avant l'ajout du peptide Aβ 1-42, montre un effet significatif et important sur le réseau de neurites aux deux plus faibles concentrations (10 nM, 100 nM) (Figure 2).

Un effet protecteur de l'acide léontopodique B1 a été observé à toutes les concentrations. Aucune mort cellulaire n'a été observée aux trois concentrations testées les plus fortes : 100 nM, 1 µM, et 10 µM (Figure 3).

Ces résultats montrent que l'acide léontopodique B1 permet une protection à la fois des neurones et des astrocytes en culture *in vitro* intoxiqués par le peptide Aβ 1-42. Un effet significatif sur le réseau de neurites a été observé pour les concentrations les plus faibles d'acide léontopodique B1. En outre, un effet significatif important a été observé sur la survie des astrocytes. Ainsi, un effet protecteur complet est observé.

### Exemple 2 : Effet de l'acide léontopodique B1 sur la prolifération d'astrocyte purs sains.

Afin de vérifier que l'effet protecteur de l'acide léontopodique B1 sur les astrocytes n'est pas le résultat d'une induction de la prolifération cellulaire, l'effet de cet acide sur la prolifération d'astrocytes sains (non traités par le peptide Aβ 1-42) a été évalué à différentes concentrations (100 nM, 1 µM, 10 µM et 50 µM).

### 2.1. Matériels et méthodes

### Culture cellulaire d'une lignée d'astrocytes

En résumé, le cerveau entier d'un embryon âgé de 15 jours de rat (Wistar) est prélevé. Le cerveau entier est dissocié par trypsinisation. Les cellules sont suspendues dans un milieu de culture du milieu DMEM (Dulbecco's modified Eagle's medium) complémenté avec 2% de pénicilline (10,000 U/mL) et d'une solution de streptomycine (10 mg/mL), et 10% de sérum de veau fœtal (FCS). Les cellules sont ensemencées à une densité de 267 000 cellules par cm² dans un flacon de culture. Au stade de la confluence cellulaire, le flacon est secoué pendant 3 heures. Le surnageant de la culture cellulaire est enlevé, les cellules sont traitées par une solution de trypsine- EDTA (Pan Biotech) à une concentration finale de 0,05% trypsine et de 0,02% EDTA. Ensuite les cellules sont congelées afin de constituer une lignée cellulaire d'astrocytes.

Les astrocytes (au stade : P4) sont ensemencées à une densité de 4 000 par puits d'une plaque de 96 puits avec de l'acide léontopodique B1 (dissous à différentes concentrations : 100 nM, 1 µM, 10 µM et 50 µM dans le milieu de culture) et sont cultivées à 37°C dans un incubateur contenant 95% d'air et 5% de CO2. Le milieu est changé tous les jours. Les astrocytes sont cultivés pendant 4 jours en présence d'acide léontopodique B1 avant de mesurer son effet sur la prolifération des astrocytes.

### Evaluation de la prolifération des astrocytes

Après quatre jours d'incubation avec l'acide léontopodique B1, un test MTT est réalisé avec le test CellTiter 96® Aqueous One Solution Cell Proliferation Assay (Promega). En résumé, 20 µL du compose tetrazolium est ajouté directement dans le puit de culture et incubé pendant 3 heures. La densité optique (DO) est mesurée à l'aide d'un spectrophotomètre (appareil Glomax, (Promega)) à une longueur d'onde de 450 nm.

### Analyse statistique

Toutes les données sont exprimées en pourcentage du contrôle des conditions (contrôle = culture d'astrocyte sans ajout d'acide léontopodique B1 = 100%). Toutes les valeurs sont exprimées en moyenne +/- l'erreur standard de la moyenne (Standard Error of the Mean SEM) (s.e.mean) de n = 6 puits par conditions. Les analyses statistiques réalisées pour les différentes conditions sont des analyses de variances ou ANOVA suivie par le test de Dunnett en utilisant le logiciel GraphPad Prism (* p < 0.05 vs condition contrôle).

### 2.2. Résultats

Aux différentes concentrations testées (de 100 nM jusqu'à 50 µM), l'acide léontopodique B1 n'induit aucun effet significatif sur la prolifération des astrocytes (Figure 4). Une légère mais non significative diminution de la concentration cellulaire a été observée à la plus forte concentration (50 µM).

### 2.3. Conclusion

L'acide léontopotique B1 rassemble les critères d'un bon candidat : protection des neurones corticaux primaires, maintien de leurs connections (c'est-à-dire l'intégrité du réseau de neurites), protection des astrocytes sans favoriser de prolifération cellulaire (potentiellement tumorale). Ainsi, l'acide léontopodique B1 présente un intérêt en tant que nouvel agent thérapeutique pour la prévention et/ou le traitement d'une maladie neurodégénérative, et en particulier la maladie d'Alzheimer.

## Revendications

1. Composés de formule générale (I) 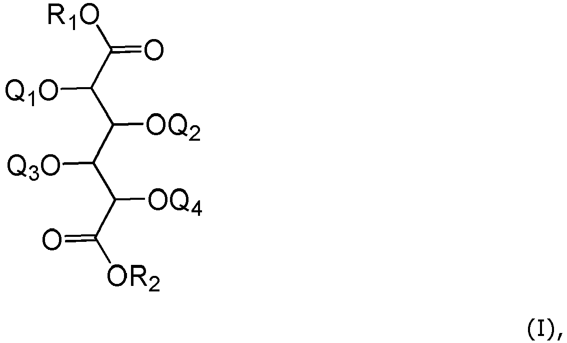 dans laquelle
R₁ et R₂ sont identiques ou différents et représentent, chacun indépendamment l'un de l'autre, un atome d'hydrogène ou un groupe alkyle en C₁-Cₚ linéaire ou ramifié, cyclique ou cyclique ramifié, saturé ou insaturé, pouvant être substitué par un ou plusieurs substituants choisis dans le groupe comprenant : -OR₃, - NR₃R₄, -NO₂, -COR₃, -COOR₃, -CF₃, -CR₃F₂, -CR₃R₄F, =O, -CN, halogène, -SR₃, - SO₂R₃ et aryle, avec
- p est un entier naturel compris entre 2 et 18,
- R₃ et R₄ sont identiques ou différents et représentent, chacun indépendamment l'un de l'autre,
- Soit un groupe choisi dans le groupe comprenant : -H, -OH, - NH₂, -NO₂, -COOH, -CF₃, -CHF₂, -CH₂F, =O, -CN, halogène, -SH et - SO₂H,
- Soit un groupe alkyle en C₁-C₆ linéaire ou ramifié, cyclique ou cyclique ramifié, saturé ou insaturé, pouvant être substitué par un ou plusieurs substituants choisis dans le groupe comprenant : -H, -OH, -NH₂, -NO₂, -COOH, -CF₃, -CHF₂, -CH₂F, =O, -CN, halogène, -SH et -SO₂H,
et
Q₁, Q₂, Q₃ et Q₄ représentent, chacun indépendamment l'un de l'autre, un radical choisi dans le groupe comprenant :
un groupe caféoyle, un groupe CH₃-CH(OH)-CH₂-CO- et un atome d'hydrogène, sous réserve qu'au moins un de ces radicaux soit un groupe caféoyle,
leurs stéréoisomères et leurs sels pharmaceutiquement ou nutraceutiquement acceptables,
pour leur utilisation dans la prévention et/ou le traitement d'une maladie neurodégénérative.

2. Composés pour leur utilisation selon la revendication 1, **caractérisés en ce que** trois quelconques des radicaux Q₁, Q₂, Q₃ et Q₄ représentent un groupe caféoyle et **en ce que** le quatrième radical représente un atome d'hydrogène.

3. Composés pour leur utilisation selon l'une quelconque des revendications 1 ou 2, **caractérisés en ce que** :
- le radical R₁ et le radical R₂ représentent, chacun indépendamment l'un de l'autre, un atome d'hydrogène ou un groupe alkyle en C₂-C_{q} avec q entier naturel compris entre 3 et 18, avantageusement un groupe alkyle en C₂-C₆ et encore plus avantageusement un groupe alkyle en C₂-C₅, éventuellement substitué par un groupe -OH, et
- trois quelconques des radicaux Q₁, Q₂, Q₃ et Q₄ représentent un groupe caféoyle et le quatrième radical représente un atome d'hydrogène.

4. Composés pour leur utilisation selon l'une quelconque des revendications 1 à 3, choisis parmi :
- les composés dans lesquels R₁ et R₂ représentent un groupe éthyle, trois quelconques des radicaux Q₁, Q₂, Q₃ et Q₄ représentent un groupe caféoyle et le quatrième radical représente un atome d'hydrogène ; et
- les composés dans lesquels R₁ et R₂ représentent un groupe 2-hydroxy-pentyle, trois quelconques des radicaux Q₁, Q₂, Q₃ et Q₄ représentent un groupe caféoyle et le quatrième radical représente un atome d'hydrogène.
- les composés dans lesquels R₁ et R₂ représentent un groupe 2-hydroxy-propyle, trois quelconques des radicaux Q₁, Q₂, Q₃ et Q₄ représentent un groupe caféoyle et le quatrième radical représente un atome d'hydrogène.
- les composés dans lesquels R₁ et R₂ représentent un groupe isopropyle, trois quelconques des radicaux Q₁, Q₂, Q₃ et Q₄ représentent un groupe caféoyle et le quatrième radical représente un atome d'hydrogène.
- les composés dans lesquels R₁ et R₂ représentent un groupe (diméthylamino)-éthyle, trois quelconques des radicaux Q₁, Q₂, Q₃ et Q₄ représentent un groupe caféoyle et le quatrième radical représente un atome d'hydrogène.
- les composés dans lesquels R₁ et R₂ représentent un groupe cinnamyle, trois quelconques des radicaux Q₁, Q₂, Q₃ et Q₄ représentent un groupe caféoyle et le quatrième radical représente un atome d'hydrogène.

5. Composés pour leur utilisation selon l'une quelconque des revendications 1 à 4, **caractérisés en ce que** ladite maladie neurodégénérative est choisie parmi : la maladie d'Alzheimer, l'ataxie spinocérébelleuse, l'atrophie multisystématisée, la maladie d'Alexander, la maladie d'Alpers, la maladie de Creutzfeldt-Jakob, la maladie de Huntington, la maladie de Parkinson, la maladie de Pick, la myofasciite à macrophages, la paralysie supranucléaire progressive, la sclérose en plaques et la sclérose latérale amyotrophique.

6. Composés de formule générale (I) 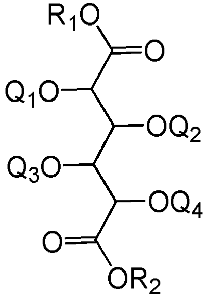 dans laquelle R₁ et R₂ sont identiques ou différents et représentent, chacun indépendamment l'un de l'autre, un atome d'hydrogène ou un groupe alkyle en C₁-Cₚ linéaire ou ramifié, cyclique ou cyclique ramifié, saturé ou insaturé, pouvant être substitué par un ou plusieurs substituants choisis dans le groupe comprenant : -OR₃, - NR₃R₄, -NO₂, -COR₃, -COOR₃, -CF₃, -CR₃F₂, -CR₃R₄F, =O, -CN, halogène, -SR₃, - SO₂R₃, aryle, avec
- p est un entier naturel compris entre 2 et 18,
- R₃ et R₄ sont identiques ou différents et représentent chacun indépendamment l'un de l'autre,
- soit un groupe choisi dans le groupe comprenant : -H, -OH, - NH₂, -NO₂, -COOH, -CF₃, -CHF₂, -CH₂F, =O, -CN, halogène, - SH et - SO₂H,
- soit un groupe alkyle en C₁-C₆ linéaire ou ramifié, cyclique ou cyclique ramifié, saturé ou insaturé, pouvant être substitué par un ou plusieurs substituants choisis dans le groupe comprenant : -H, -OH, -NH₂, -NO₂, -COOH, -CF₃, -CHF₂, -CH₂F, =O, -CN, halogène, -SH et -SO₂H,
sous réserve qu'au moins un des radicaux R₁ ou R₂ ne soit pas un atome d'hydrogène,
et
Q₁, Q₂, Q₃ et Q₄ représentent chacun, indépendamment l'un de l'autre, un radical choisi dans le groupe comprenant : un atome d'hydrogène, un groupe caféoyle et un groupe CH3- CH(OH)- CH₂CO-, sous réserve qu'au moins un de ces radicaux soit un groupe caféoyle,
à l'exception des composés suivants :
• 1-O-méthyle ester de 2,3,4-tricaféoyle d'acide altrarique
• 1-O-méthyle ester de 2,3-dicaféoyle d'acide altrarique leurs stéréoisomères et leurs sels pharmaceutiquement ou nutraceutiquement acceptables.

7. Composés selon la revendication 6, **caractérisés en ce que** trois quelconques des radicaux Q₁, Q₂, Q₃ et Q₄ représentent un groupe caféoyle et **en ce que** le quatrième radical représente un atome d'hydrogène.

8. Composés selon l'une quelconque des revendications 6 ou 7, destinés à être utilisés comme médicament.

9. Composition pharmaceutique comprenant au moins un composé selon l'une quelconque des revendications 6 ou 7, et au moins un excipient pharmaceutiquement acceptable, ou composition nutraceutique comprenant au moins un composé selon l'une quelconque des revendications 6 ou 7, et au moins un excipient nutraceutiquement acceptable.

10. Composition pharmaceutique selon la revendication 9 pour son utilisation dans la prévention et/ou le traitement d'une maladie neurodégénérative, notamment la maladie d'Alzheimer.

## Patentansprüche

1. Verbindungen mit der allgemeinen Formel (I) 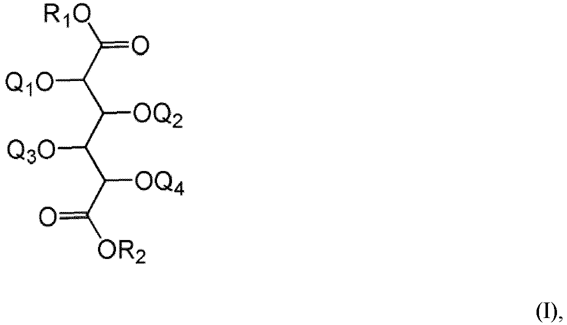 in der
R₁ und R₂ gleichartig oder verschiedenartig sind und, jeweils unabhängig voneinander, für ein Wasserstoffatom oder eine geradkettige oder verzweigte, zyklische oder zyklisch-verzweigte C₁-Cₚ-Alkylgruppe gesättigter oder ungesättigter Beschaffenheit stehen, die mit einem oder mehreren Substituenten versehen sein kann, die ausgewählt ist aus der Gruppe umfassend: -OR₃, -NR₃R₄, -NO₂, -COR₃, -COOR₃, -CF₃, -CR₃F₂, -CR₃R₄F, =O, -CN, Halogen, -SR₃, - SO₂R₃ und Aryl, wobei
- p eine ganze natürliche Zahl im Bereich von 2 bis 18 ist,
- R₃ und R₄ gleichartig oder verschiedenartig sind und, jeweils unabhängig voneinander, für Folgendes stehen
entweder eine Gruppe, die ausgewählt ist aus der Gruppe umfassend: -H, -OH, -NH₂, -NO₂, -COOH, -CF₃, -CHF₂, -CH₂F, =O, -CN, Halogen, -SH und - SO₂H,
oder eine geradkettige oder verzweigte, zyklische oder zyklisch-verzweigte C₁-C₆-Alkylgruppe gesättigter oder ungesättigter Beschaffenheit, die mit einem oder mehreren Substituenten versehen sein kann, die ausgewählt ist aus der Gruppe umfassend: -H, -OH, -NH₂, -NO₂, -COOH, -CF₃, -CHF₂, -CH₂F, =O, -CN, Halogen, -SH und -SO₂H,
und
Q₁, Q₂, Q₃ und Q₄, jeweils unabhängig voneinander, für ein Radikal stehen, das ausgewählt ist aus der Gruppe umfassend:
eine Caffeoylgruppe, eine CH₃-CH(OH)-CH₂-CO- Gruppe und ein Wasserstoffatom, mit der Maßgabe, dass es sich bei mindestens einem dieser Radikale um eine Caffeoylgruppe handelt, deren Stereoisomere und deren pharmazeutisch oder nutrazeutisch unbedenkliche Salze, für eine Verwendung derselben in der Vorbeugung und/oder Behandlung einer neurodegenerativen Krankheit.

2. Verbindungen zur Verwendung derselben nach Anspruch 1,
**dadurch gekennzeichnet, dass**
drei beliebige der Radikale Q₁, Q₂, Q₃ und Q₄ für eine Caffeoylgruppe stehen, und dadurch, dass das vierte Radikal für ein Wasserstoffatom steht.

3. Verbindungen zur Verwendung derselben nach einem der Ansprüche 1 oder 2,
**dadurch gekennzeichnet, dass:**
- das Radikal R₁ und das Radikal R₂, jeweils unabhängig voneinander, für ein Wasserstoffatom oder eine C₂-C_{q}-Gruppe stehen, wobei q eine natürliche ganze Zahl im Bereich von 3 bis 18 ist, vorteilhafterweise für eine C₂-C₆-Alkylgruppe und auf noch vorteilhaftere Weise für eine C₂-C₅-Alkylgruppe, die möglicherweise mit einer OH-Gruppe substituiert ist, und
- drei beliebige der Radikale Q₁, Q₂, Q₃ und Q₄ für eine Caffeoylgruppe stehen und das vierte Radikal für ein Wasserstoffatom steht.

4. Verbindungen zur Verwendung derselben nach einem der Ansprüche 1 bis 3, die ausgewählt sind aus:
- den Verbindungen, in denen R₁ und R₂ für eine Ethylgruppe stehen, drei beliebige der Radikale Q₁, Q₂, Q₃ und Q₄ für eine Caffeoylgruppe stehen und das vierte Radikal für ein Wasserstoffatom steht; und
- den Verbindungen, in denen R₁ und R₂ für eine 2-Hydroxypentylgruppe stehen, drei beliebige der Radikale Q₁, Q₂, Q₃ und Q₄ für eine Caffeoylgruppe stehen und das vierte Radikal für ein Wasserstoffatom steht.
- den Verbindungen, in denen R₁ und R₂ für eine 2-Hydroxypropylgruppe stehen, drei beliebige der Radikale Q₁, Q₂, Q₃ und Q₄ für eine Caffeoylgruppe stehen und das vierte Radikal für ein Wasserstoffatom steht.
- den Verbindungen, in denen R₁ und R₂ für eine Isopropylgruppe stehen, drei beliebige der Radikale Q₁, Q₂, Q₃ und Q₄ für eine Caffeoylgruppe stehen und das vierte Radikal für ein Wasserstoffatom steht.
- den Verbindungen, in denen R₁ und R₂ für eine (Dimethylamino)ethylgruppe stehen, drei beliebige der Radikale Q₁, Q₂, Q₃ und Q₄ für eine Caffeoylgruppe stehen und das vierte Radikal für ein Wasserstoffatom steht.
- den Verbindungen, in denen R₁ und R₂ für eine Cinnamylgruppe stehen, drei beliebige der Radikale Q₁, Q₂, Q₃ und Q₄ für eine Caffeoylgruppe stehen und das vierte Radikal für ein Wasserstoffatom steht.

5. Verbindungen zur Verwendung derselben nach einem der Ansprüche 1 bis 4,
**dadurch gekennzeichnet, dass**
die neurodegenerative Krankheit aus den folgenden ausgewählt ist: der Alzheimer-Krankheit, der spinozerebellären Ataxie, der Multisystematrophie, der Alexander-Krankheit, dem Alpers-Huttenlocher-Syndrom, der Creutzfeldt-Jakob-Krankheit, der Chorea Huntington, der Parkinson-Krankheit, der Pick-Krankheit, der Makrophagischen Myofasciitis, der progressiven supranukleären Blickparese, der Multiplen Sklerose und der Amyotrophen Lateralsklerose.

6. Verbindungen mit der allgemeinen Formel (I) 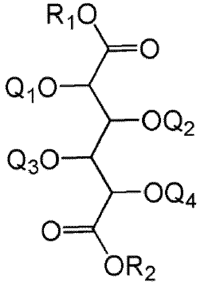 in der
R₁ und R₂ gleichartig oder verschiedenartig sind und, jeweils unabhängig voneinander, für ein Wasserstoffatom oder eine geradkettige oder verzweigte, zyklische oder zyklisch-verzweigte C₁-Cₚ-Alkylgruppe gesättigter oder ungesättigter Beschaffenheit stehen, die mit einem oder mehreren Substituenten versehen sein kann, die ausgewählt ist aus der Gruppe umfassend: -OR₃, - NR₃R₄, -NO₂, -COR₃, -COOR₃, -CF₃, -CR₃F₂, -CR₃R₄F, =O, -CN, Halogen, -SR₃, - SO₂R₃, Aryl, wobei
- p eine ganze natürliche Zahl im Bereich von 2 bis 18 ist,
- R₃ und R₄ gleichartig oder verschiedenartig sind und, jeweils unabhängig voneinander, für Folgendes stehen
- entweder eine Gruppe, die ausgewählt ist aus der Gruppe umfassend: -H, -OH, -NH₂, -NO₂, -COOH, -CF₃, -CHF₂, -CH₂F, =O, -CN, Halogen, -SH und - SO₂H,
- oder eine geradkettige oder verzweigte, zyklische oder zyklisch-verzweigte C₁-C₆-Alkylgruppe gesättigter oder ungesättigter Beschaffenheit, die mit einem oder mehreren Substituenten versehen sein kann, die ausgewählt ist aus der Gruppe umfassend: -H, -OH, -NH₂, -NO₂, -COOH, -CF₃, -CHF₂, -CH₂F, =O, -CN, Halogen, -SH und -SO₂H,
mit der Maßgabe, dass es sich bei mindestens einem der Radikale R₁ und R₂ nicht um ein Wasserstoffatom handelt,
und
Q₁, Q₂, Q₃ und Q₄, jeweils unabhängig voneinander, für ein Radikal stehen, das ausgewählt ist aus der Gruppe umfassend: ein Wasserstoffatom, eine Caffeoylgruppe und eine CH₃-CH(OH)-CH₂CO-Gruppe, mit der Maßgabe, dass es sich bei mindestens einem dieser Radikale um eine Caffeoylgruppe handelt,
mit Ausnahme der folgenden Verbindungen:
• 2,3,4-Tricaffeoyl-1-O-methylester von Altrarsäure
• 2,3-Dicaffeoyl-1-O-methylester von Altrarsäure
deren Stereoisomere und deren pharmazeutisch oder nutrazeutisch unbedenkliche Salze.

7. Verbindungen nach Anspruch 6,
**dadurch gekennzeichnet, dass**
drei beliebige der Radikale Q₁, Q₂, Q₃ und Q₄ für eine Caffeoylgruppe stehen und dadurch, dass das vierte Radikal für ein Wasserstoffatom steht.

8. Verbindungen nach einem beliebigen der Ansprüche 6 oder 7, die für die Verwendung als Arzneimittel bestimmt sind.

9. Pharmazeutische Zusammensetzung, umfassend mindestens eine Verbindung nach einem beliebigen der Ansprüche 6 oder 7 sowie mindestens einen pharmazeutisch unbedenklichen Hilfsstoff, oder nutrazeutische Zusammensetzung, umfassend mindestens eine Verbindung nach einem beliebigen der Ansprüche 6 oder 7 sowie mindestens einen nutrazeutisch unbedenklichen Hilfsstoff.

10. Pharmazeutische Zusammensetzung nach Anspruch 9, zur Verwendung derselben in der Vorbeugung und/oder Behandlung einer neurodegenerativen Krankheit, insbesondere der Alzheimer-Krankheit.

## Claims

1. Compounds of the general formula (I) 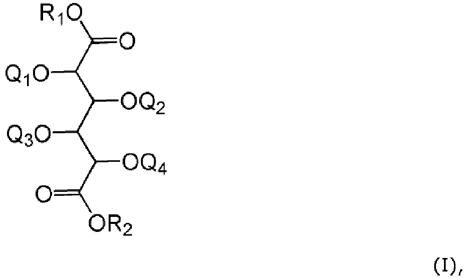 wherein
R₁ and R₂ are identical or different and represent, each independently of each other, a hydrogen atom or a linear or branched, cyclic or cyclic branched, saturated or unsaturated C₁-Cₚ alkyl group, which can be substituted with one or more substituents selected from the group comprising: -OR₃, -NR₃R₄, -NO₂, -COR₃, -COOR₃, -CF₃, -CR₃F₂, -CR₃R₄F, =O, -CN, halogen, -SR₃, -SO₂R₃ and aryl, with
- p is an integer between 2 and 18,
- R₃ and R₄ are identical or different and represent, each independently of each other,
- either a group selected from the group comprising: -H, -OH, -NH₂, -NO₂, -COOH, -CF₃, -CHF₂, -CH₂F, =O, -CN, halogen, -SH and -SO₂H,
- or a linear or branched, cyclic or cyclic branched, saturated or unsaturated C₁-C₆ alkyl group, which can be substituted with one or more substituents selected from the group comprising: -H, -OH, -NH₂, -NO₂, -COOH, -CF₃, -CHF₂, -CH₂F, =0, -CN, halogen, -SH and -SO₂H,
Q₁, Q₂, Q₃ and Q₄ represent, each independently of each other, a radical selected from the group comprising:
a cafeoyl group, a CH₃-CH(OH)-CH₂-CO- group and a hydrogen atom, with the proviso that at least one of these radicals is a cafeoyl group, stereoisomers thereof and pharmaceutically or nutraceutically acceptable salts thereof,
for their use in preventing and/or treating a neurodegenerative disease.

2. The compounds for their use according to claim 1, **characterised in that** any three of radicals Q₁, Q₂, Q₃ and Q₄ represent a cafeoyl group and **in that** the fourth radical represents a hydrogen atom.

3. The compounds for their use according to any of claims 1 or 2, **characterised in that**:
- radical R₁ and radical R₂ represent, each independently of each other, a hydrogen atom or a C₂-C_{q} alkyl group with q being an integer between 3 and 18, advantageously a C₂-C₆ alkyl group and still more advantageously a C₂-C₅ alkyl group, optionally substituted with an -OH group, and
- any three of radicals Q₁, Q₂, Q₃ and Q₄ represent a cafeoyl group and the fourth radical represents a hydrogen atom.

4. The compounds for their use according to any of claims 1 to 3, selected from:
- compounds in which R₁ and R₂ represent an ethyl group, any three of radicals Q₁, Q₂, Q₃ and Q₄ represent a cafeoyl group and the fourth radical represents a hydrogen atom; and
- compounds in which R₁ and R₂ represent a 2-hydroxy-pentyl group, any three of radicals Q₁, Q₂, Q₃ and Q₄ represent a cafeoyl group and the fourth radical represents a hydrogen atom,
- compounds in which R₁ and R₂ represent a 2-hydroxy-propyl group, any three of radicals Q₁, Q₂, Q₃ and Q₄ represent a cafeoyl group and the fourth radical represents a hydrogen atom,
- compounds in which R₁ and R₂ represent an isopropyl group, any three of radicals Q₁, Q₂, Q₃ and Q₄ represent a cafeoyl group and the fourth radical represents a hydrogen atom,
- compounds in which R₁ and R₂ represent a (dimethylamino)-ethyl group, any three of radicals Q₁, Q₂, Q₃ and Q₄ represent a cafeoyl group and the fourth radical represents a hydrogen atom,
- compounds in which R₁ and R₂ represent a cinnamyl group, any three of radicals Q₁, Q₂, Q₃ and Q₄ represent a cafeoyl group and the fourth radical represents a hydrogen atom.

5. The compounds for their use according to any of claims 1 to 4, **characterised in that** said neurodegenerative disease is selected from: Alzheimer disease, spinocerebellar ataxia, multiple system atrophy, Alexander disease, Alpers disease, Creutzfeldt-Jakob disease, Huntington's disease, Parkinson's disease, Pick's disease, macrophage myofascitis, progressive supranuclear palsy, multiple sclerosis and amyotrophic lateral sclerosis.

6. The compounds of the general formula (I) 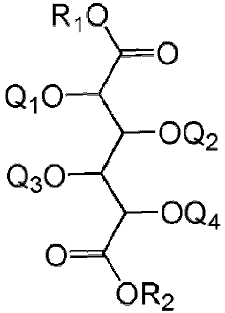 wherein
R₁ and R₂ are identical or different and represent, each independently of each other, a hydrogen atom or a linear or branched, cyclic or cyclic branched, saturated or unsaturated C₁-Cₚ alkyl group, which can be substituted with one or more substituents selected from the group comprising: -OR₃, -NR₃R₄, -NO₂, -COR₃, -COOR₃, -CF₃, -CR₃F₂, -CR₃R₄F, =0, -CN, halogen, -SR₃, -SO₂R₃, aryl, with
- p is an integer between 2 and 18,
- R₃ and R₄ are identical or different and represent, each independently of each other,
- either a group selected from the group comprising: -H, -OH, -NH₂, -NO₂, -COOH, -CF₃, -CHF₂, -CH₂F, =O, -CN, halogen, -SH and -SO₂H,
- or a linear or branched, cyclic or cyclic branched, saturated or unsaturated C₁-C₆ alkyl group, which can be substituted with one or more substituents selected from the group comprising: -H, -OH-, -NH₂, -NO₂, -COOH, -CF₃, -CHF₂, -CH₂F, =0, -CN, halogen, -SH and -SO₂H,
with the proviso that at least one of radicals R₁ and R₂ is not a hydrogen atom,
and
Q₁, Q₂, Q₃ and Q₄ each represent, independently of each other, a radical selected from the group comprising: a hydrogen atom, a cafeoyl group, and a CH3-CH(OH)-CH₂CO-group, with the proviso that at least one of these radicals is a cafeoyl group,
except for the following compounds:
• 2,3,4-tricafeoyl altraric acid 1-0-methyl ester
• 2,3,dicafeoyl altraric acid 1-O-methyl ester
stereoisomers thereof and pharmaceutically or nutraceutically acceptable salts thereof.

7. The compounds according to claim 6, **characterised in that** any three of radicals Q₁, Q₂, Q₃ and Q₄ represent a cafeoyl group and **in that** the fourth radical represents a hydrogen atom.

8. The compounds according to any of claims 6 or 7, for their use as a drug.

9. A pharmaceutical composition comprising at least one compound according to any of claims 6 or 7, and at least one pharmaceutically acceptable excipient, or a nutraceutical composition comprising at least one compound according to any of claims 6 or 7, and at least one nutraceutically acceptable excipient.

10. The pharmaceutical composition according to claim 9 for their use in preventing and/or treating a neurodegenerative disease, especially Alzheimer disease.
